# EUROPEAN PATENT SPECIFICATION

(11) **EP 3 554 476 B1**
(45) Date of publication and mention of the grant of the patent: **13.04.2022**
(21) Application number: 17829291.8
(22) Date of filing: 14.12.2017
(51) Int. Cl.: A61K 47/10, A61K 47/12, A61K 47/26, A61K 9/08, A61K 31/185, A61K 49/00, G01N 33/50

(54) **OPHTHALMIC SOLUTION OF LISSAMINE GREEN AND ITS USE IN OPHTHALMOLOGY**
OPHTHALMISCHE LÖSUNG VON LISSAMIN GREEN UND DEREN VERWENDUNG IN DER OPHTHALMOLOGIE
SOLUTION OPHTALMIQUE DE VERT DE LISSAMINE ET SON UTILISATION EN OPHTALMOLOGIE

(30) Priority: 15.12.2016 IT 201600127037
(43) Date of publication of application: 23.10.2019
(73) Proprietor: Oftalab Srl, 20135 Milano (IT)
(72) Inventor: BARABINO, Stefano, 20135 Milano (IT)
(74) Representative: Villa, Livia
(86) International application number: PCT/IB2017/057935
(87) International publication number: WO 2018/109704

(56) References cited:
- WO-A1-2014/098888
- CN-A- 103 301 478
- BRON A J ET AL: "Clinical staining of the ocular surface: Mechanisms and interpretations", PROGRESS IN RETINAL AND EYE RESEARCH, OXFORD, GB, vol. 44, 23 October 2014 (2014-10-23), pages 36-61, XP029115577, ISSN: 1350-9462, DOI: 10.1016/J.PRETEYERES.2014.10.001
- GORRINGE ET AL: "Lissamine green as a protein stain in paper electrophoresis", CLINICA CHIMICA ACTA, ELSEVIER BV, AMSTERDAM, NL, vol. 2, no. 5, 1 October 1957 (1957-10-01) , pages 353-371, XP025848643, ISSN: 0009-8981, DOI: 10.1016/0009-8981(57)90031-1 [retrieved on 1957-10-01]

## Description

### FIELD OF INVENTION

The present invention relates to an ophthalmic solution of lissamine green which has shown to be particularly stable over time and well tolerated by the eye. Therefore, the ophthalmic solution finds advantageous application in the diagnosis of corneal and conjunctival anomalies.

### STATE OF THE ART

Lissamine green is an organic acidic dye, which is produced synthetically; its staining properties are very similar to those obtained when using rose bengal. However, differently from rose bengal, lissamine green is painless and offers a better colour contrast to vessels and bleeding. Lissamine green selectively stains suffering and dehydrated cells, thus guaranteeing observation of the conjunctival epithelium and highlighting abraded or desquamated epithelial areas.

Lissamine green is typically used, therefore, in vital staining of the external eye surface, not only for the diagnosis of Sjögrens syndrome, which, among many symptoms, also causes ocular dryness (xerophthalmia) characterised by keratoconjunctivitis, but also for detecting ocular dryness due to environmental or age factors or to the use of contact lenses, or for the examination of conjunctival irritations of various origins.

CN 103301478 A discloses aqueous solutions for vital staining of the eye, comprising a mixture of sodium fluorescein and lissamine green.

At present, diagnostic tests employing lissamine green are performed by using cardboard strips on one end of which lissamine green solution has been dried. At the time of use, the end of the strip bearing the dye is irrorated with one or two drops of physiological solution, which serves to redissolve the lissamine green. The drop is then let drop into the patient's eye and stains the damaged epithelial areas green.

Said strips are designed to overcome the problem of the reduced water solubility of lissamine green and the consequent high instability of aqueous solutions of lissamine green. The aforesaid strips allow to provide doses of dye in solid form, which are then brought back to a solution form only at the time of use and only in the necessary volume, i.e. one or two drops.

However, these strips show certain drawbacks of a practical nature, as well as of technical nature.

Indeed, since the drop of dye is often released through direct contact between the strip and the eye surface, from the patient's viewpoint, this creates considerable discomfort, in addition to local pain, especially if the surface of the eye is damaged as a result of any of the aforesaid diseases.

Moreover, precisely because lissamine green is not completely water-soluble, the extemporaneous addition of one or two drops thereof to the dry dye does not achieve a constant and measurable concentration of the said dye before use.

The object of the present invention is therefore to offer a practical and effective alternative, which is also well tolerated by patients, to the strips currently in use.

### SUMMARY OF INVENTION

Said object has been achieved by an ophthalmic aqueous solution having pH of 4.8-5.1 and comprising 0.01 to 0.3% w/v of alkyl polyglucosides, as a stabilizing agent, 1.4-1.8% w/v of lissamine green, 1.5-2.5% w/v of citric acid-sodium citrate buffer, and water, wherein the concentrations of lissamine green and stabilizing agent are in a ratio of 200:1 to 2:1.

For the purposes of the present invention, "aqueous solution" is understood as a homogeneous mixture of the various components dissolved in water.

In another aspect, the present invention relates to said aqueous solution for use in the vital staining of the external eye surface.

The characteristics and advantages of the present invention shall become readily apparent from the following detailed description and from the embodiments provided by way of illustrative, non-limiting examples, as the aqueous solution according to the invention has surprisingly shown high stability over time and a notable ease of use.

### DETAILED DESCRIPTION OF THE INVENTION

Therefore, the invention relates to an aqueous ophthalmic solution having pH of 4.8-5.1 and comprising 0.01 to 0.3% w/v of alkyl polyglucosides, as a stabilizing agent, 1.4-1.8% w/ v of lissamine green, 1.5-2.5% w/v of citric acid-sodium citrate buffer, and water, wherein the concentrations of lissamine green and stabilizing agent are in a ratio of 200:1 to 2:1.

For the purposes of the present specification, the expression "ophthalmologically acceptable non-ionic surfactant" is understood as a non-ionic surfactant suitable for ophthalmologic administration, which is therefore compatible with tissues and fluids present on the eye surface.

Ophthalmologically acceptable non-ionic surfactants are alkyl polyglucosides, octyl glucoside, decyl maltoside, fatty alcohols, cetyl alcohol, oleyl alcohol, cocamide MEA, cocamide DEA, cocamide TEA, C10-C22 alkyl ethoxylates, fatty acid esters of polyols, esters of fatty acid amine oxide, and mixtures thereof. C10-C22 ethoxylated alkyls (e.g., polyoxyl 40 stearate, Myrj 52, Brij 30, Tween 20, Tween 40, Tween 60, Tween 65, Tween 80, polyoxyl 40 hydrogenated castor oil) are preferably chosen from ethoxylates, diethoxylates, triethoxylates, tetraethoxylates, pentaethoxylates or hexaethoxylates of C10-C22 fatty acids, C10-C22 fatty acid amides, C10-C22 fatty amides, C10-C22 alcohols, C10-C22 alkyl phenols, and C10-C22 fatty acid esters of polyols, wherein the hydrogen atom of the terminal hydroxyl group may be, optionally, substituted by a hydrophobic moiety, such as a benzyl, butyl, or methyl group, or mixtures thereof. The fatty acid esters of polyols (e.g., sorbitan monolaurate, sorbitan monooleate) are preferably chosen from C10-C22 fatty acids esters of glycerol, sorbitol, saccharose, polyglucoside, or a mixture thereof. The esters of fatty acid amine oxides are preferably chosen from esters of C10-C22 alkyl dimethyl amine oxides, C10-C22 alkyl diethanolamine oxides, and mixtures thereof.

For the purposes of the present invention, the compositions comprise alkyl polyglucosides as non-ionic surfactant.

It has surprisingly been found that the aqueous solution having the characteristics stated above shows a remarkable stability over time, since both the lissamine green titre and the pH remain satisfactory for at least 18 months. Furthermore, an aqueous solution with a predetermined concentration offers the further advantage of being administrable to the eye surface in exactly the same way as eye drops, thereby overcoming the drawbacks observed with the use of cardboard strips.

Indeed, the patient will no longer have the discomfort ascribable to the contact of the end of the cardboard strip with the eye surface, nor will there be any more diagnostic uncertainties ascribable to the variability of the concentration of lissamine green in the drops extemporaneously prepared on the cardboards.

Said stabilizing agent is in a concentration of 0.01 to 0.3 wt% of the volume of the aqueous solution.

For the purposes of the present invention, "wt% of the volume" or "% w/v" is understood as "grams per 100 ml of aqueous solution".

More preferably, said stabilizing agent is in a concentration of up to 1 wt% of the volume of the aqueous solution.

The lissamine green is in a concentration of 1.4 to 1.8 wt% of the volume of the aqueous solution.

The pH of the aqueous solution is 4.8-5.1.

In the aqueous solution according to the invention, the buffer serves to maintain the chosen pH.

The buffer is in a concentration of 1.5-2.5 wt% of the volume of the aqueous solution.

The buffer is citric acid-sodium citrate.

In particularly preferred embodiments, the lissamine green is the only active ingredient for the vital staining of the external eye surface which is present in the aqueous solution. In further particularly preferred embodiments of the aqueous solution according to the invention, water is the only solvent present therein.

In still further particularly preferred embodiments, the aqueous solution according to the invention does not comprise preservatives or others dyes in addition to lissamine green.

The lissamine green is in a higher concentration than the stabilizing agent.

The concentrations of lissamine green and stabilizing agent are in a ratio of 200:1 to 2:1, more preferably 50:1 to 5:1, and even more preferably 30:1 to 10:1.

In further embodiments, the aqueous solution according to the invention is in the form of a unit dose.

Preferably, said unit dose comprises 0.1-2.0 ml of aqueous solution as disclosed above. In particularly preferred embodiments, said unit dose comprises 0.2-1.0 ml of aqueous solution as disclosed above.

It should be understood that the preferred aspects specified for the individual components of the aqueous solution, should likewise be considered as preferred in the unit dose embodiments stated above.

The aqueous solution according to the invention, also in the form of a unit dose, may further comprise ophthalmologically acceptable excipients. The term "ophthalmologically acceptable excipient" should be understood as a compound or mixture which is suitable for use in a formulation for administration to the external eye surface. For example, an excipient of this type should generally not cause an adverse response in a patient, nor should it significantly inhibit the action of the lissamine green on the eye surface.

Suitable excipients are antioxidants, gelling agents, sequestrants, binders, lubricants, thickening agent, tonicity regulators, filmogenic substances, and mixtures thereof. Thus, suitable antioxidants are sodium metabisulphite, sodium thiosulphate, ascorbic acid, sodium ascorbate, glucose, cysteine, and mixtures thereof.

Suitable sequestrants are EDTA and monosodium, disodium, and potassium salts thereof, diethylene triamine pentamethylene phosphonic acid, hexamethylene diamine tetramethylene phosphonic acid, ethylene diamine tetramethylene phosphonic acid, amino trimethylene phosphonates and mixtures thereof.

Preferably, the sequestrants are EDTA, monosodium, disodium, and potassium salts thereof, and mixtures thereof.

Suitable thickening agents are TS polysaccharide, PVP, carboxyvinyl polymers, xanthan gum, sodium hyaluronate, hydroxyethyl cellulose (HEC), hydroxypropyl methylcellulose (HPMC), polyethylene glycol, mucopolysaccharides, cellulose and the esters thereof, natural gums and the esters thereof, pectins, polyacrylates, and mixtures thereof. Preferably, the thickening agents are TS polysaccharide, mucopolysaccharides, and mixtures thereof.

Tonicity regulators are inorganic salts, such as sodium chloride, potassium chloride, magnesium chloride, and calcium chloride, or polyols or sugars, such as glycerol, propylene glycol, erythritol, mannitol, sorbitol, and trehalose, or amino acids, such as carnitine and betaine.

The aqueous solution according to the invention has a pH of 4.8-5.1 and comprises:
- 0.01 to 0.3% w/v of alkyl polyglucosides,
- 1.4-1.8% w/v of lissamine green,
- 1.5-2.5% w/v of citric acid-sodium citrate buffer, and
- water.

In more preferred embodiments, the aqueous solution according to the invention has a pH of 4.8-5.1 and comprises:
- 0.01 to 0.3% w/v of alkyl polyglucosides,
- 1.4-1.8% w/v of lissamine green,
- 1.5-2.5% w/v of citric acid-sodium citrate buffer,
- 0.01-0.5% w/v of sodium metabisulphite,
- 0.01-0.2% w/v of EDTA,
- 0.01-0.3% w/v of TS polysaccharide, and
- water.

In some embodiments, the aqueous solution according to the invention has a pH of 4.8-5.1 and consists essentially of lissamine green, citric acid-sodium citrate buffer, buffer, water, and a stabilizing agent, said stabilizing agent being alkyl polyglucosides. The expression "consisting essentially of" means that the lissamine is the only active ingredient for the vital staining of the external eye surface which is present in the aqueous solution according to the invention, while further components or excipients do not interfere with the action of the lissamine green and are water-miscible and soluble.

In other embodiments, the aqueous solution according to the invention has a pH of 4.8-5.1 and consists of lissamine green, citric acid-sodium citrate buffer, water, and a stabilizing agent, said stabilizing agent being alkyl polyglucosides, and ophthalmologically acceptable excipients.

The aqueous solution according to the present invention may be prepared by using known methods. Indeed, for administration to the external eye surface, the components may, for example, be mixed as they are or with one or more excipients, added one after the other, under stirring.

Typically, the aqueous solutions of the present invention are sterilised prior to use according to known methods, e.g. by a gamma ray treatment.

In another aspect, the present invention relates to the aqueous solution disclosed above for use in the vital staining of the external eye surface. As already mentioned, indeed, the aqueous solution according to the invention has surprisingly overcome the drawbacks associated with the use of cardboard strips bearing dried lissamine green.

Therefore, the aqueous solution according to the invention is therefore usable in the ophthalmic field as it is well tolerated by the eye and therefore finds advantageous application in the diagnosis of corneal and conjunctival anomalies.

Preferably, the aqueous solution according to the invention is administered to the external eye surface in an amount of 0.005-0.050 ml, preferably 0.010-0.015 ml.

It should be understood that all the possible combinations of the preferred aspects of the components of the aqueous solution, the unit doses, the preparation, and the uses of said aqueous solution are described herein and therefore are also preferred.

It should also be understood that all aspects identified as favourable and advantageous for the aqueous solution and the components thereof, should be deemed equally preferable and advantageous also for the preparation and the uses of said aqueous solution.

Below are working examples of the present invention provided for illustrative purposes.

### EXAMPLES

### Example 1.

The following aqueous solution with a pH of 4.9 was prepared:

| | % w/v |
|---|---|
| Lissamine green | 1.65% |
| Tribasic sodium citrate | 1.36% |
| Citric acid | 0.50% |
| TRITON^{™} CG-50 (^{∗}) | 0.10% |
| Distilled water | balance to 100% |

| | |
|---|---|
| (^{∗}) alkyl-polyglucoside non-ionic surfactant (Dow Chemical Company) | |

### Reference Example 2.

Two aqueous solutions were prepared for comparison:

| | A | B |
|---|---|---|
| | % w/v | % w/v |
| Lissamine green | 1.50% | 1.50% |
| Tribasic sodium citrate | 0.50% | 0.50% |
| Citric acid | 0.03% | 0.03% |
| Distilled water | balance to 100% | balance to 100% |
| pH | 5.5 | 6.5 |

### Example 3.

The following aqueous solution with a pH of 4.9 was prepared:

| | % w/v |
|---|---|
| Lissamine green | 1.65% |
| Tribasic sodium citrate | 1.36% |
| Citric acid | 0.50% |
| Sodium metabisulphite | 0.20% |
| EDTA | 0.05% |
| TRITON^{™} CG-50 | 0.10% |
| TS polysaccharide | 0.10% |
| Distilled water | balance to 100% |

### Reference Example 4.

The following aqueous solution with a pH of 5.0 was prepared:

| | % w/v |
|---|---|
| Lissamine green | 1.50% |
| Tribasic sodium citrate | 0.50% |
| Citric acid | 0.04% |
| Sodium chloride | 0.40% |
| Sodium metabisulphite | 0.20% |
| EDTA | 0.05% |
| TRITON^{™} CG-50 | 0.10% |
| TS polysaccharide | 0.1% |
| Distilled water | balance to 100% |

### Reference Example 5.

The following aqueous solution with a pH of 5.0 was prepared:

| | % w/v |
|---|---|
| Lissamine green | 1.5% |
| Tribasic sodium citrate | 0.5% |
| Citric acid | 0.04% |
| Sodium chloride | 0.3% |
| Sodium metabisulphite | 0.2% |
| EDTA | 0.05% |
| Triton | 0.1% |
| Trehalose | 1.0% |
| PVP | 3.0% |
| Distilled water | balance to 100% |

### Example 6.

### Test of stability over time

The aqueous solutions A and B used for comparison in Reference Example 2 were tested with the same composition but different pHs.

Samples of both solutions were subjected to two separate stability studies:
- the first set of samples of solutions A and B was stored at 25°C (RH 60%),
- the second set of samples of solutions A and B was stored at 40°C (RH 74%).

After six months in the conditions stated above, all the samples showed a severe decline in the green lissamine titre, accompanied by a progressive acidification and the solutions, which were originally an intense blue colour, tended towards green.

Samples of the aqueous solution in Example 1 were subjected to a stability study for a total of nine months at 25°C (RH 60%).

After the first two months, during which time a decrease of approximately 10-12% was observed in the lissamine green titre, the said titre then remained nearly stable until the end of the observation period.

The pH also showed a similar trend, with a decrease of approximately 6% in absolute value over the first two months, followed by a stable level until the end of the observation period.

## Claims

1. An ophthalmic aqueous solution having pH of 4.8-5.1 and comprising:
- 0.01 to 0.3% w/v of alkyl polyglucosides, as a stabilizing agent,
- 1.4-1.8% w/v of lissamine green,
- 1.5-2.5% w/v of citric acid-sodium citrate buffer, and
- water,
wherein the concentrations of lissamine green and stabilizing agent are in a ratio of 200:1 to 2:1.

2. The aqueous solution of claim 1, wherein lissamine green is the only active ingredient for the vital staining of the external eye surface which is present in the aqueous solution.

3. The aqueous solution of claim 1 or 2, where water it is the only solvent present therein.

4. The aqueous solution of any one of claims 1-3, wherein said aqueous solution does not comprise preservatives or other dyes in addition to lissamine green.

5. The aqueous solution of any one of claims 1-4, wherein the concentrations of lissamine green and stabilizing agent are in a ratio of 50:1 to 5:1, preferably 30:1 to 10:1.

6. The aqueous solution of any one of claims 1-5, being in the form of a unit dose comprising 0.1-2.0 ml of the aqueous solution.

7. The aqueous solution of claim 6, wherein said unit dose comprises 0.2-1.0 ml of aqueous solution.

8. The aqueous solution of any one of claims 1-7, having a pH of 4.8-5.1 and comprising:
- 0.01 to 0.3% w/v of alkyl polyglucosides,
- 1.4-1.8% w/v of lissamine green,
- 1.5-2.5% w/v of citric acid-sodium citrate buffer,
- 0.01-0.5% w/v of sodium metabisulphite,
- 0.01-0.2% w/v of EDTA,
- 0.01-0.3% w/v of TS polysaccharide, and
- water.

9. The aqueous solution of any one of claims 1-8, further comprising ophthalmologically acceptable excipients selected from antioxidants, gelling agents, sequestrants, binders, lubricants, thickening agent, tonicity regulators, filmogenic substances, and mixtures thereof.

10. The aqueous solution of any one of claims 1-9 for use in the vital staining of the external eye surface.

11. The aqueous solution for use of claim 10, to be administered to the external eye surface in an amount of 0.005-0.050 ml, preferably 0.010-0.015 ml.

## Patentansprüche

1. Ophthalmische wässrige Lösung, die pH von 4,8-5,1 aufweist und Folgendes umfasst:
- 0,01 bis 0,3 Gew.-Vol.-% Alkylpolyglucoside als Stabilisierungsmittel,
- 1,4-1,8 Gew.-Vol.-% Lissamingrün,
- 1,5-2,5 Gew.-Vol.-% Citronensäure-Natriumcitrat-Puffer, und
- Wasser,
wobei die Konzentrationen von Lissamingrün und Stabilisierungsmittel in einem Verhältnis von 200:1 bis 2:1 sind.

2. Wässrige Lösung nach Anspruch 1, wobei Lissamingrün der einzige Wirkstoff für die Vitalfärbung der äußeren Augenoberfläche ist, der in der wässrigen Lösung vorhanden ist.

3. Wässrige Lösung nach Anspruch 1 oder 2, wobei Wasser das einzige Lösungsmittel ist, das darin vorhanden ist.

4. Wässrige Lösung nach einem der Ansprüche 1-3, wobei die wässrige Lösung keine Konservierungsmittel oder anderen Farbstoffe zusätzlich zu Lissamingrün umfasst.

5. Wässrige Lösung nach einem der Ansprüche 1-4, wobei die Konzentrationen von Lissamingrün und Stabilisierungsmittel in einem Verhältnis von 50:1 bis 5:1, bevorzugt 30:1 bis 10:1 sind.

6. Wässrige Lösung nach einem der Ansprüche 1-5, die in der Form einer Einheitsdosis ist, die 0,1-2,0 ml der wässrigen Lösung umfasst.

7. Wässrige Lösung nach Anspruch 6, wobei die Einheitsdosis 0,2-1,0 ml wässrige Lösung umfasst.

8. Wässrige Lösung nach einem der Ansprüche 1-7, die einen pH von 4,8-5,1 aufweist und Folgendes umfasst:
- 0,01 bis 0,3 Gew.-Vol.-% Alkylpolyglucoside,
- 1,4-1,8 Gew.-Vol.-% Lissamingrün,
- 1,5-2,5 Gew.-Vol.-% Citronensäure-Natriumcitrat-Puffer,
- 0,01-0,5 Gew.-Vol.-% Natriummetabisulfit,
- 0,01-0,2 Gew.-Vol.-% EDTA,
- 0,01-0,3 Gew.-Vol.-% TS-Polysaccharid, und
- Wasser.

9. Wässrige Lösung nach einem der Ansprüche 1-8, ferner umfassend ophthalmologisch annehmbare Hilfsstoffe, ausgewählt aus Antioxidantien, Geliermitteln, Maskierungsmitteln, Bindemitteln, Schmiermitteln, Verdickungsmittel, Tonizitätsregulatoren, filmbildenden Substanzen und Mischungen davon.

10. Wässrige Lösung nach einem der Ansprüche 1-9 zur Verwendung bei der Vitalfärbung der äußeren Augenoberfläche.

11. Wässrige Lösung zur Verwendung nach Anspruch 10 zur Verabreichung an die äußere Augenoberfläche in einer Menge von 0,005-0,050 ml, bevorzugt 0,010-0,015 ml.

## Revendications

1. Solution aqueuse ophtalmique présentant un pH de 4,8 à 5,1 et comprenant :
- 0,01 à 0,3 % en p/v de polyglucosides d'alkyle, en tant qu'agent stabilisant,
- 1,4 à 1,8 % en p/v de vert de lissamine,
- 1,5 à 2,5 % en p/v de tampon acide citrique-citrate de sodium, et
- de l'eau,
les concentrations de vert de lissamine et d'agent stabilisant étant dans un rapport de 200:1 à 2:1.

2. Solution aqueuse selon la revendication 1, ledit vert de lissamine étant le seul ingrédient actif pour la coloration vitale de la surface externe de l'œil qui est présent dans la solution aqueuse.

3. Solution aqueuse selon la revendication 1 ou 2, l'eau étant le seul solvant présent dans celle-ci.

4. Solution aqueuse selon l'une quelconque des revendications 1 à 3, ladite solution aqueuse ne comprenant pas de conservateurs ou d'autres colorants en plus du vert de lissamine.

5. Solution aqueuse selon l'une quelconque des revendications 1 à 4, lesdites concentrations de vert de lissamine et d'agent stabilisant étant dans un rapport de 50:1 à 5:1, de préférence de 30:1 à 10:1.

6. Solution aqueuse selon l'une quelconque des revendications 1 à 5, étant sous la forme d'une dose unitaire comprenant 0,1 à 2,0 ml de la solution aqueuse.

7. Solution aqueuse selon la revendication 6, ladite dose unitaire comprenant 0,2 à 1,0 ml de solution aqueuse.

8. Solution aqueuse selon l'une quelconque des revendications 1 à 7, présentant un pH de 4,8 à 5,1 et comprenant :
- 0,01 à 0,3 % en p/v de polyglucosides d'alkyle,
- 1,4 à 1,8 % en p/v de vert de lissamine,
- 1,5 à 2,5 % en p/v de tampon acide citrique-citrate de sodium,
- 0,01 à 0,5 % en p/v de métabisulfite de sodium,
- 0,01à 0,2 % en p/v d'EDTA,
- 0,01 à 0,3 % en p/v de polysaccharide de TS, et
- de l'eau.

9. Solution aqueuse selon l'une quelconque des revendications 1 à 8, comprenant en outre des excipients ophtalmologiquement acceptables choisis parmi les antioxydants, les agents gélifiants, les agents séquestrants, les liants, les lubrifiants, les agents épaississants, les régulateurs de tonicité, les substances filmogènes et leurs mélanges.

10. Solution aqueuse selon l'une quelconque des revendications 1 à 9 pour une utilisation dans la coloration vitale de la surface externe de l'œil.

11. Solution aqueuse pour une utilisation selon la revendication 10, destinée à être administrée à la surface externe de l'œil en une quantité de 0,005 à 0,050 ml, de préférence de 0,010 à 0,015 ml.
